# EUROPEAN PATENT SPECIFICATION

(11) **EP 0 714 601 B1**
(45) Date of publication and mention of the grant of the patent: **30.08.2000**
(21) Application number: 95402643.1
(22) Date of filing: 23.11.1995
(51) Int. Cl.: A01N 43/40, A01N 53/00, A01N 25/16

(54) **Residual control of parasites by long-acting shampoo formulations**
Dauerhafte Kontrolle von Parasiten mittels langwirkender Shampoo-Formulierungen
Contrôle résiduel des parasites avec des formulations de shampooing à longue durée

(30) Priority: 28.11.1994 US 345526
(43) Date of publication of application: 05.06.1996
(73) Proprietor: VIRBAC, 06516 Carros (FR)
(72) Inventor: Miller, Thomas A., Carrollton, Texas 75010 (US)
(74) Representative: Orès, Bernard

(56) References cited:
- EP-A- 0 352 529
- EP-A- 0 445 924
- EP-A- 0 542 078
- EP-A- 0 623 337
- WO-A-86/03374
- WO-A-91/15953
- FR-A- 2 696 318

## Description

The present invention relates to the use of a residue forming concentration of certain compounds comprising synthetic pyrethroid insecticides or certain insect growth regulator compounds, especially certain nitrogen containing heterocyclics with residual control of ectoparasites on homoisothermic or warm-blooded animals by their application in long acting shampoo formulations, and in other embodiment by the combination in said shampoo formulations of both synthetic pyrethroid insecticide and insect growth regulator at a concentration such that each has the ability to resist post shampooing washout from the animal's haircoat.

Bloodsucking ectoparasites of the order Insecta include such as *Ctenocephalides felis* and *Ctenocephalides canis* (cat and dog fleas), as well as lice, mosquitos, tabanids, tsetse and other biting flies, and Acarina such as *Boophilus, Amblyomma, Anocentor, Dermacentor, Haemaphysalis, Hyalomma, Ixodes, Rhipicentor, Margaropus, Rhipicephalus, Argas, Otobius* and *Ornithodoros* (ticks) and the like, infest or attack many useful homoisothermic animals including farm animals such as cattle, swine, sheep, goats, poultry such as chicken, turkeys and geese, fur bearing animals such as mink, foxes, chinchilla, rabbits and the like, and pet animals such as dogs and cats. Other than for lice, these ectoparasites spend a major portion of their life cycle off the host in its environment. Control measures thus must have at least 3 objectives. One is to kill existing parasites on an already infested host, the second is to control or prevent successful reinfestation by new parasites from the environment and the third is to control or prevent development of the off-host, free living stages of the parasites' life cycles in the environment.

Ticks are described as hard ticks or soft ticks and are characterized as one host, two host, or three host ticks. They attach to a suitable host animal and feed on blood and body fluids. Engorged females detach and drop from the host and lay large numbers of eggs (2,000 to 20,000) in a suitable niche in the ground or in some other sheltered location in which hatching occurs. The larva then seek a host from which to obtain a blood meal. Larvae of one host ticks molt on the host twice to become nymphs and adults without leaving the host. Larvae of two and three host ticks drop off the host, molt in the environment and find a second or third host (as nymph or adult) on which to feed.

Ticks are responsible for the transmission and propagation of many human and animal diseases throughout the world. Ticks of major economic importance include *Boophilus, Rhipicephalus, Ixodes, Hyalomma, Amblyomma* and *Dermacentor.* They are vectors of bacterial, viral, rickettsial and protozoal diseases and cause tick paralysis and tick toxicosis. Even a single tick can cause paralysis consequent to injecting its saliva into its host in the feeding process. Tick-borne diseases are usually transmitted by multiple-host ticks. Such diseases, including Babesiosis, Anaplasmosis, Theileriosis and Heart Water are responsible for the death and/or debilitation of vast numbers of pet and food animals throughout the world. In many temperate countries, Ixodid ticks transmit the agent of a chronic, debilitating disease, Lyme disease, from wildlife to man. In addition to disease transmission, ticks are responsible for great economic losses in livestock production. Losses are attributable not only to death, but also to damage of hides, loss of growth, reduction in milk production and reduced grade of meat. Although the debilitating effects of tick infestations on animals have been recognized for years and tremendous advances have been made in tick control programs, no entirely satisfactory methods for controlling or eradicating these parasites have been forthcoming. Ticks have often developed resistance to chemical toxicants and dependent control measures.

Infestation of pets by fleas has long been a nuisance to pet owners. Because fleas are able to survive and their eggs and larvae to develop, pupate and emerge as new fleas under a wide range of environmental conditions, controlling flea infestation requires a multifaceted program that must be vigorously applied to achieve any measure of success. Adult fleas live in the coat of the cat or dog and feed on blood. Male and female fleas mate, still in the animal's coat, and the female flea lays her eggs, which fall off and are distributed to the animal's environment. By this mechanism, while the total environment of the pet animal is infested with flea eggs, infestation is greatest in locations where the pet spends most of its time. Eggs hatch to larvae in about two days. There are three larval stages, each lasting about three days. In the last stage, the larva spins a cocoon and transforms into a pupa. Under optimum conditions (i.e., 33°C and 65% relative humidity), eggs develop through larvae to pupae in about 8-10 days. After a further period of approximately 8 days, the pupae develop into young adult fleas in the cocoon, still dispersed in the pet's environment. These pre-emerged adult fleas wait in their pupae until they sense, by carbon dioxide tension and/or vibrations, the presence of an animal host, and then emerge explosively and jump into the air and onto the passing host.

Under suitable environmental conditions of temperature and humidity, unfed emerged fleas that fail to find a host can survive for some time in the environment, waiting for a suitable host. It takes at least three weeks for eggs to develop to pre-emerged adults, able to reinfest a host animal. However, the pre-emerged adults can remain viable in the cocoon for months, as long as one year. In addition, under sub-optimal temperature conditions, it can take 4-5 months for eggs to develop into pupae containing pre-emerged adults. Fleas require a blood meal in order to become sexually mature and able to reproduce. After their first blood meal, they undergo a shift in metabolism such that they cannot survive for any time off the host. The blood must come from the correct animal and the female flea's appetite requires that it consumes as much as 5 times its body weight of blood each day. The long life cycle, and especially the extended period of pre-emergence dormancy, has made flea control with compounds applied topically to pet animals and their environment difficult and not entirely satisfactory. Most active ingredients when applied topically to the pet and to its environment have a limited residual effect, thus reinfestation by newly-emerged adults from the pet's environment is a constant problem.

Infestation of dogs and cats with fleas has several undesirable effects for the animals and their owners. Such undesirable effects include local irritation and annoying itching, leading to scratching. A high proportion of pet animals become allergic to flea saliva, resulting in the chronic condition known as flea bite allergy (or flea allergy). This condition causes the animal to bite and scratch, leading to excoriation of the skin, secondary pyogenic infection, hair loss and chronic severe inflammatory skin changes. Furthermore, most dogs and cats that are infested with fleas also become infected with *Dipylidium caninum,* the tapeworm transmitted by fleas.

In prolonged absence of a suitable host, newly emerged fleas attack any mammal, including humans, although they are not capable of full reproductive potential if human blood is their sole source of nutrition. Even in the presence of the pet animal, the owner may be bitten by fleas. Some humans may suffer allergic skin diseases as a result of being bitten by dog and cat fleas.

Since, like most insects, fleas can adapt to survive exposure to normally toxic agents, and the tolerance of dogs and cats to chemical agents varies, it is desirable to have a multiplicity of agents and methods available for controlling fleas. Prior art methods have included numerous toxic agents such as organophosphates (e.g., chlorpyrifos), carbamates (e.g., carbaryl), pyrethroids (e.g., natural pyrethrins, permethrin and related synthetic pyrethroids), and other topical insecticides formulated and designed to kill the adult flea after their application to the pet. Many of the effective residual action toxic agents against fleas, such as DDT, benzene hexachloride and other chlorinated hydrocarbon insecticides, have been banned from most countries because of environmental persistence of residues and their effect on certain wildlife. Others have been banned because of long-term health risks, including risks of cancer to chronically exposed humans. In the United States, currently approved and available toxic agents that are effective against fleas, some only briefly, will always be under scrutiny because of concerns for long-term health hazards to pets and to their owners. These considerations have limited utility of insecticidal and acaricidal toxic compounds for control of fleas and ticks on pet animals and of ectoparasites on animals in general.

One very commonly used measure for control of existing infestations of fleas and ticks on pet animals is by applying shampoo containing quick acting non-residual insecticides and acaricides, usually natural pyrethrins and related short acting synthetic pyrethroids. While these active ingredients are usually effective in killing most of the existing ectoparasites on the host, rinsing off as required for all shampoos removes the active ingredients and leaves the animal immediately susceptible to reinfestation with new ectoparasites from its environment. Absence of residual effect is, therefore, a universally acknowledged feature of pesticidal shampoos. It has heretofore been necessary, after shampooing, to immediately apply another treatment system, such as a dip or sponge-on concentrate, a spray, a foam, a powder or a collar containing stable and residual active ingredients to provide protection to the animal against reinfestation by new ectoparasites. Multiple treatment procedures, starting with an insecticidal shampooing followed by a dipping or spraying are inconvenient, time consuming and such repeated manipulation is annoying to the pet animal, which often is resistant or antagonistic to further handling. Further, in addition to killing ectoparasites, a major objective of shampooing a pet animal is to clean the haircoat so as to leave it soft, manageable and cosmetically desirable. Application after shampooing (as needed for residual control of ectoparasites) of dips, sponge-ons, sprays, powders or foam insecticidal products often contain spreading oils or silicones and therefore leave the haircoat in a cosmetically undesirable condition since it may be sticky, greasy or oily. In the case of solvent-based residual insecticide sprays, the skin may be irritated and dried out which then leads to the need to apply further dermatologic treatments such as emollients and moisturizers, that may further contribute to an undesirable appearance of the haircoat.

There is consequently an overwhelming need for a convenient single method of treatment that would firstly kill existing ectoparasites and then, through long-acting residual insecticidal or growth regulator or ovicidal activity, further prevent ectoparasite reinfestations and ablate the fertility of any new ectoparasite, and yet leave the haircoat clean, lustrous, soft, manageable and cosmetically pleasing to the owner. Due to the variability of toxic effects from multiple topical applications of toxicant insecticides in various animal species, and the high dose rates required for long lasting residual activity with many of the prior art compounds, it is desirable that additional alternative control agents be made available.

Because the detergent action of a shampoo generally removes oil soluble materials from the haircoat, it has heretofore been believed that insecticidally active ingredients in shampoos are rinsed from the haircoat when such materials are applied as shampoo formulations. Even when long lasting residual effects were observed with spray formulation of synthetic pyrethroids, no such long lasting residual control activity has been noted for insecticidal, growth regulating or ovicidal shampoo formulations. For example, Allan and the present inventor found that the combination of liquid silicones with pyrethroids, including permethrin, extended the useful life of formulations and in spray on formulations demonstrated long lasting residual effects, in U.S. Patent 4,668,666. Although shampoo formulations are disclosed in U.S. Patent 4,668,666, there was no recognition or teaching that at large enough rates a residue forming concentration of pyrethroids could be reached which would provide a residual acting shampoo. Indeed the advantages of the invention of U.S. Patent 4,668,666 included "stabilized, nontoxic pesticidal compositions whereby the presence of a liquid alkyl aryl silicone stabilizer permits the use of very small amounts of active ingredients (insecticide, synergists and repellents) while still providing long-term killing action, thereby permitting low-cost manufacture of the compositions". (Allan and Miller, U.S. Patent 4,668,666, col. 4, lines 55 to 62).

Matthewson U.S. Patents 4,404,223 and 4,940,729 disclose synthetic pyrethroids in various formulations including shampoos, but do not disclose a method or composition for applying pyrethroids at a sufficient concentration to achieve a long-acting residual concentration in the haircoat. Use of insect growth regulators in shampoo formulation is less common, and as with toxic agents, there are no reports of long-acting residue producing insect growth regulating shampoo compositions or methods of treating parasites by use of long acting shampoo compositions.

Certain substituted heterocyclic of known insecticidal activity are disclosed in U.S. Patents 4,970,222, 4,879,292 and 4,751,223. However, while these juvenile hormone-like nitrogen containing heterocyclic compounds have been shown to be effective when applied topically to the pet and its environment, as disclosed in Alig et al., U.S. Patent 5,057,527, the complete formulation had to be left deposited on the substrate (pet animal's coat or its environment) without wash off. Post-application rinsing is generally known to diminish the activity by removing the active compounds with the wash water. Therefore, these juvenile hormone-like nitrogen containing heterocyclic compounds have not heretofore been suggested as being resistant to wash off or to provide residual efficacy applied by shampoo.

The present invention relates to the use of a detergent and of at least one active compound selected from the nitrogen containing heterocyclic compounds of the formula: 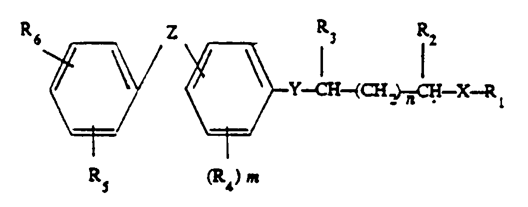 wherein :
- R₁ is one of the following groups : 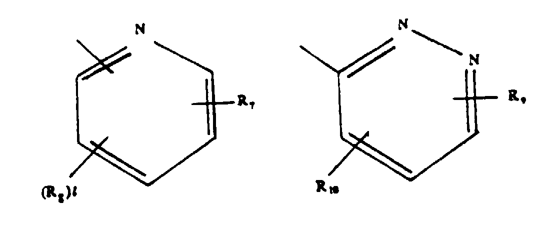 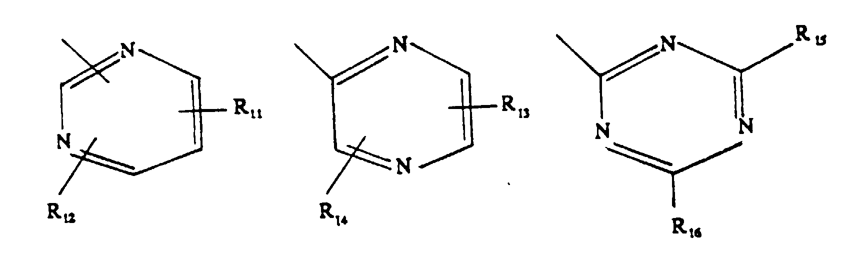 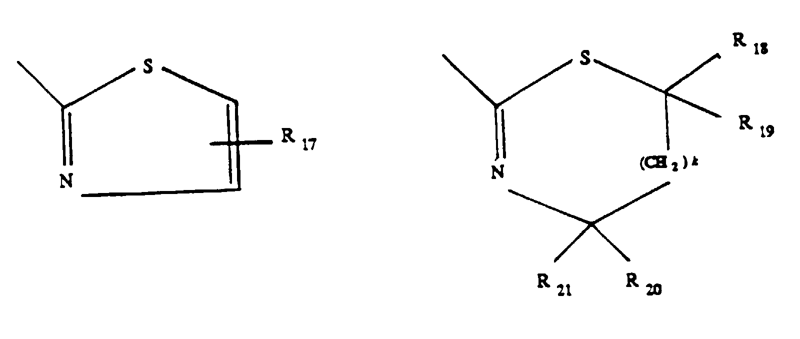 in which R₇, R₈, R₉, R₁₀, R₁₁, R₁₂, R₁₃, R₁₄, R₁₅, R₁₆ and R₁₇ are, the same or different, each a hydrogen atom, a halogen atom, a C₁-C₄ alkoxy group, a C₁-C₄ alkylthio group, a trifluoro methyl group or a nitro group ; R₁₈, R₁₉, R₂₀ and R₂₁ are, the same or different, each a hydrogen atom or a methyl group, *k* is an integer of 0 to 1 and *l* is an integer of 0 to 3 ;
- R₂ and R₃ are, the same or different, each a hydrogen atom, a halogen atom or a methyl group ;
- R₄ is a halogen atom or a methyl group ;
- R₅ and R₆ are, the same or different, each a hydrogen atom, a halogen atom, a C₁-C₄ haloalkyl group or a C₁-C₄ haloalkoxy group ;
- X, Y and Z are, the same or different, each an oxygen atom, a sulfur atom or a methylene group, *m* is an integer of 0 to 4 and *n* is an integer of 0 to 2 ; for the preparation of a long-acting composition for the residual control of ectoparasites which attack warm-blooded animals, said composition being under the form of a shampoo designed to be rinsed out, and comprising a dose of active compound sufficient for leaving on the haircoat of said warm-looded animals, after rinsing out, an ovicidally residual effective amount of said active compound.

Indeed, it has surprisingly been found that, when administered topically in sufficient concentration in a shampoo formulation that is then thoroughly washed out, the various compounds of the invention demonstrate a powerful residual ovicidal effect towards ectoparasites.

As used herein, the term ectoparasite has its normal meaning in the art and includes fleas, ticks, lice, mosquitos, tabanids, tsetse and other biting flies, and especially the species named above.

In a first preferred embodiment of the invention, the active compound is a nitrogen containing heterocyclic compound in which R₁ is a 2-substituted pyridine.

Advantageously, the active compound is a (4-phenoxyphenoxy)ethoxy pyridine and more preferably the pyriproxifen, 2-[1-methyl-2-(4-phenoxyphenoxy)ethoxy] pyridine (commercially available from the Sumitomo Chemical Company or from McLaughlin Gormley, King Co. under the trademark NYLAR®).

In this first embodiment, the dose of the ovicidally active compound in the shampoo is conveniently in the range from 0.001% to 5%, more preferably from 0.005% to 2.5% and most preferably from 0.1% to 0.5%.

In another preferred embodiment of the invention, the shampoo composition further comprises an insecticidally and acaracidally active compound which is a synthetic pyrethroid selected from the group consisting of permethrin, cypermethrin, cyhalothrin, lambdacyhalothrin, deltamethrin, tralomethrin, cyfluthrin, flucythrin, flumethrin, fluvalinate, fenvalerate or mixtures thereof, and leaves on the haircoat of the warm-blooded animals an ovicidally residual effective amount of said nitrogen containing heterocyclic compound and an insecticidally and acaricidally residual effective amount of said synthetic pyrethroid.

The dose of the synthetic pyrethroid is conveniently in the range from 0.2 to 5.0%, more preferably from 0.5 to 3.0% and most preferably from 1.0 to 2.0%.

Advantageously, the nitrogen containing heterocyclic compound is the 2-[1-methyl-2-(4-phenoxyphenoxy)ethoxy] pyridine (pyriproxifen) whereas the synthetic pyrethroid is the permethrin.

In such an embodiment, the dose of the nitrogen containing heterocyclic compound in the shampoo composition is conveniently in the range from 0.001% to 5% whereas the dose of the synthetic pyrethroid in the shampoo composition is in the range from 0.2% to 5.0%.

Preferably, the dose of the nitrogen containing heterocyclic compound in the shampoo composition is in the range from 0.005% to 2.5% whereas the dose of the synthetic pyrethroid in the shampoo composition is in the range from 0.5% to 3.0%.

More preferably, the dose of the nitrogen containing heterocyclic compound in the shampoo composition is in the range from 0.01% to 0.5% whereas the dose of the synthetic pyrethroid in the shampoo composition is in the range from 1.0% to 2.0%.

Whatever the embodiment, the shampoo composition can countain from 1.0% to 20.0% emulsifier in combination with 10% to 60% detergent as well as one or more components selected from the group consisting of emollients, fragances, preservatives and viscosity controlling agents.

Topically-applied insecticides and acaricides, including the synthetic pyrethroids (e.g., permethrin and related compounds), which are effective against existing ectoparasites when applied in shampoos are well known in the art. Many are also known by residually effective when applied in, for instance, sprays, dips, sponge-ons, sprays, foams, powders or collars since the active ingredients are left on the animal coat and not washed out. Heretofore the art has not provided shampoo formulations of synthetic pyrethroid insecticides, acaricides, or insect growth regulators that have been reported to show residual activity when washed out after being applied in a shampoo formulation. In the commercial market there exist shampoos containing synthetic pyrethroids (i.e., permethrin at 0.1%) that although to some degree immediately insecticidally and acaricidally effective, do not provide long lasting residual insecticidal or acaricidal activity against reinfestation of the pet after rinse off. Therefore the concept that application of permethrin in a shampoo formulation might have residual activity after rinse out has not heretofore been proposed since there have been no label or advertising claims of residual activity for shampoos. Further, the level of synthetic pyrethroid used in existing shampoos is below the level needed to confer residual insecticidal activity after rinse off.

A particularly convenient method of removing existing infestation, of preventing reinfestation by ectoparasites and of preventing environmental build-up of potential new ectoparasites is the combination in a single shampoo of a residue producing amount of an ovicidally active nitrogen containing heterocyclic compound such as pyriproxifen, in combination with a residue producing amount of a synthetic pyrethroid (including but not limited to permethrin, cypermethrin, cyhalothrin, lambdacyhalothrin, deltamethrin, tralomethrin, cyfluthrin, flucythrin, flumethrin, fluvalinate, fenvalerate), which shampoo is rinsed out of the animal's haircoat yet provides extended, long-acting residual insecticidal and acaricidal efficacy against reinfestation by new ectoparasites from the animal's environment. Therefore, even after the residual insecticidal and acaricidal activities of the insecticide-acaricide that survived rinse out have been exhausted, new ectoparastites that may succeed in parasitizing the animal and survive are sterilized, the life cycle of the ectoparasite is effectively interrupted for a long period of time and environmental accumulation and development of new ectoparasites to reinfest the animal is prevented by this prevention of fertile ectoparasite eggs.

According to the present invention, existing ectoparasitic insects and acarines are killed by the toxicant active ingredient when the shampoo is applied. After rinsing out the shampoo formulation, new ectoparasites are exposed in the haircoat for an extended residual period to effective toxicant residues of the nitrogen containing heterocyclic compound. The active ovicidal compound may be administered in shampoos containing from 0.001% to 5%, more preferably from 0.005% to 2.5% and most preferably from 0.01% to 0.5%. Advantageously, the ovicidal compound in these amounts may be combined with insecticidal and acaricidal synthetic pyrethroid agents, including but not limited to permethrin, cypermethrin, deltamethrin, cyhalothrin, lambdacyhalothrin, tralomethrin, cyfluthrin, flucythrin, flumethrin, fluvalinate, fenvalerate, formulated in the shampoo in the range of from 0.2% to 5%, more preferably from 0.5% to 3.0% and most preferably from 1.0% to 2%.

The shampoo prepared according to the present invention can be used in a method of topically preventing the infestation of dogs and cats by fertile fleas capable of reproducing and hence of contamining the dogs' and cats' environment with eggs, larvae and new fleas, which method comprises shampooing said dogs or cats with shampoo formulations, that are designed to be rinsed out, containing an ovicidally effective amount of a compound of the formula : 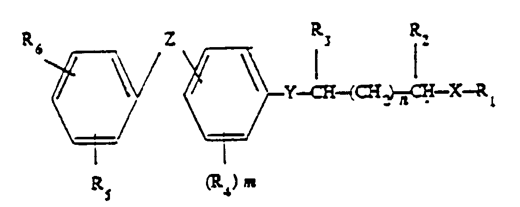 wherein :
- R₁ is one of the following groups : 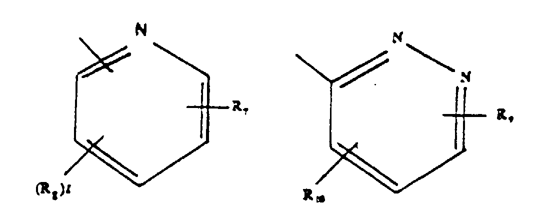 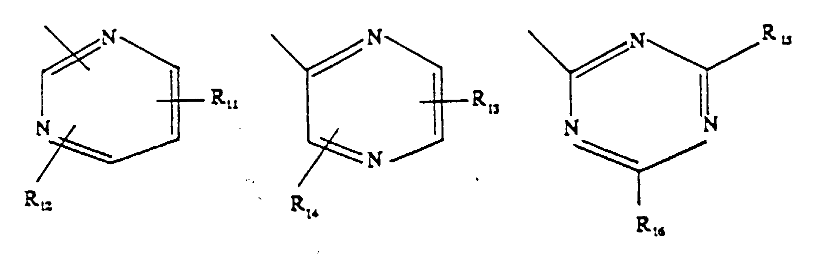 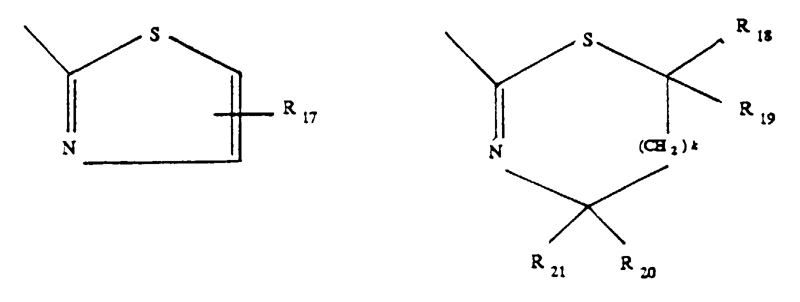 in which R₇, R₈, R₉, R₁₀, R₁₁, R₁₂, R₁₃, R₁₄, R₁₅, R₁₆ and R₁₇ are, the same or different, each a hydrogen atom, a halogen atom, a C₁-C₄ alkoxy group, a C₁-C₄ alkylthio group, a trifluoro methyl group or a nitro group ; R₁₈, R₁₉, R₂₀ and R₂₁ are, the same or different, each a hydrogen atom or a methyl group, *k* is an integer of 0 to 1 and *l* is an integer of 0 to 3 ;
- R₂ and R₃ are, the same or different, each a hydrogen atom, a halogen atom or a methyl group ;
- R₄ is a halogen atom or a methyl group ;
- R₅ and R₆ are, the same or different, each a hydrogen atom, a halogen atom, a C₁-C₄ haloalkyl group or a C₁-C₄ haloalkoxy group ;
- X, Y and Z are, the same or different, each an oxygen atom, a sulfur atom or a methylene group, *m* is an integer of 0 to 4 and *n* is an integer of 0 to 2 ; the post-rinse residue of which is transmitted to the ectoparasite as it crawls in the haircoat of the animal that has been treated with a shampoo containing the ovicidal compound.

In another aspect, the shampoo prepared according to the present invention can be used in a method of preventing the propagation of fleas comprising the application to the pet animal of a shampoo formulation designed to be rinsed off which contains an effective amount of ovicidal compound and more more specifically the provision after rinse out from the haircoat, of an effective residual amount of ovicidal compound to the host animal's haircoat. The ovicidal compound is conveniently applied in the shampoo formulation at a rate of 0.001% to 5%, preferably 0.005% to 2.5% and most preferably 0.01% to 0.5%. Shampooing may usefully be repeated at regular intervals convenient to the pet owner and as need to maintain a clean, cosmetically acceptable haircoat. Shampoos containing the higher levels of the ovicidal compound will provide ovicidally effective residual levels of active compound in the haircoat for periods in excess of three months. Such ovicidal efficacy may be somewhat diminished but will still be effective for long periods, even if the animal's haircoat were, for cleanliness and cosmetic reasons, to be subsequently shampooed with a cosmetic but non-insecticidal, non-acaricidal, non-ovicidal shampoo.

According to the present invention, the ovicidally effective heterocyclic compound is not applied in pure form, but in the form of a shampoo composition which, in addition to containing the active ingredient, contains solvents, co-solvents or emulsifiers to solubilize the active ingredients, detergents to clean the haircoat and skin, emollients, dermal nutrients and detanglers to soften and condition the skin and haircoat, fragrances to leave the haircoat with a pleasing odor and to suppress natural odors from the pet's skin, preservatives to prevent microbial growth in the formulation, thickeners and viscosity control agents to provide a shampoo of manageable viscosity, pH adjusters and water, which are tolerated by the host animal. Commercial products will normally be formulated at a strength ready for use by the end user but may also be provided in a concentrate to be diluted by the end user with water prior to application to the pet's coat.

Materials known from veterinary practice to be suitable for formulation of shampoos and for application to the pet animal may be employed as formulation assists. A number of examples are cited below. Suitable solvents and co-solvents such as alcohols, natural or synthetic oils and glycol ethers may be employed to solubilize the active ingredients. Emulsifiers, preferably of the less irritant non-ionic class, may be employed to emulsify the active ingredients, solvents, co-solvents, emollients and fragrances in the water base of the formulation. Detergents of the type commonly used in shampoo formulations for application to animal hair and skin, such as laurates and lauryl sulfates, alkanolamides and cocamide salts and esters, will be incorporated for the purposes of removing dirt, grime, natural oils, dandruff and debris from the haircoat. Foam builders to provide a luxurious foaming action expected by users may be included. Emollients and skin nutrients designed to provide a healthy skin and a cosmetically desirable haircoat may also be included. Such compounds may include oat protein, lanolin, glycerin, chitosan, alginates, essential fatty acids, vitamins and alpha hydroxy natural acids. Preservatives to inhibit microbial growth in the formulation may include, singly or in mixtures, benzoates, methyl- and propyl parabens and formaldehyde donors, such as dimethylhydantions. Fragrances designed to leave a pleasant residual aroma on the haircoat may be of a floral or herbal type and formulations may also contain quaternary ammonium compounds as coat detanglers and for natural animal odor suppression. Viscosity adjusters may include sodium chloride that acts on anionic detergents to increase viscosity, and other compounds such as colloidal clays and cellulose esters designed to improve viscosity and feel of the shampoo. pH adjusters may include mild natural acids such as lactic, citric, malic acids and hydroxides and bicarbonates.

In a preferred embodiment, insecticidally and acaricidally effective halogen containing synthetic pyrethroids are combined with nitrogen containing heterocyclics of the present invention to produce novel, multifunction shampoo compositions for immediate control of existing ectoparasites and, after rinsing out, residual insecticidal and acaricidal activity against new ectoparasites and further extended ovicidal activity against new ectoparasites. The insecticidal and acaricidal halogen containing synthetic pyrethroids include, but are not limited to, permethrin, cypermethrin, cyhalothrin, lambdacyhalothrin, deltamethrin, tralomethrin, flucythrin, cyfluthrin, flumethrin, fluvalinate, fenvalerate, of which the effective formulations strength will be in the range of 0.2% to 5%, preferably 0.5% to 3.0% and most preferably 1% to 2%. Especially preferred combinations are an juvenile hormone or ovicidally effective amount of methoprene, phenoxy carb, a benzoyl phenyl urea, or pyriproxifen or a nitrogen containing heterocyclic as defined above in combination with an insecticidally and acaricidally effective amount of a synthetic pyrethroid, especially a halogen containing synthetic pyrethroid. Each such active ingredient may be used individually or in combination within the ranges set out above. The materials may also be used in combination with other antiparasitic agents such as avermectin or ivermectin. Another especially preferred composition comprises concentrations sufficient to leave an ovicidally effective residual amount of pyriproxifen, singly or in combination with an insecticidal and acaricidally effective residual amount of synthetic pyrethroid in a pharmaceutically acceptable shampoo formulation designed to be rinsed out of the animal's haircoat after lathering.

The shampoo prepared according to the invention can furthermore be used in a method which comprises shampooing a warm-blooded animal with a composition comprising at a concentration sufficient an ovicidally effective amount of pyriproxifen in combination with an insecticidally and acaricidally effective amount of a synthetic pyrethroid selected from the group comprising the group comprising permethrin, cypermethrin, cyhalothrin, lambdacyhalothrin, deltamethrin, tralomethrin, cyfluthrin, flucythrin, flumethrin, fluvalinate, fenvalerate, such that the active ingredients effectively kill existing parasites at the time of shampooing and, after rinsing, leave in the haircoat ovicidally effective amounts of pyriproxifen and insecticidally and acaricidally effective amounts of synthetic pyrethroid sufficient to protect the animal from reinfestation by ectoparasites and further to sterilize, for an even longer period of time, ectoparasites that may survive the insecticidal and acaricidal activity of the synthetic pyrethroid.

A preferred embodiment of this method comprises the steps of applying an insecticidally and acaricidally effective dose of synthetic pyrethroid and an ovicidally effective dose of pyriproxifen in a shampoo formulation comprising a mixture of 0.001% to 5% of pyriproxifen in a combination with 0.2% to 0.5% of synthetic pyrethroid to a warm blooded animal for subsequent rinse out, preferably to a dog, cat, cow, sheep, goat, pig, mink, fox, rabbit, chicken, duck or goose such that the active ingredients effectively kill existing parasites at the time of shampooing and after rising leave in the haircoat ovicidally effective amounts of pyriproxifen and insecticidally and acaricidally effective amounts of synthetic pyrethroid sufficient to protect the animal from reinfestation by ectoparasites and further to sterilize for an even longer period of time, ectoparasites that may survive the insecticidal and acaricidal activity of the synthetic pyrethroid. The compositions comprising the nitrogen containing heterocyclic ovicide and the insecticidally and acaricidally effective synthetic pyrethroid of the present invention, especially the preferred composition of the combination of 0.005% to 2.5% of pyriproxifen with 0.5% to 3.0% of the synthetic pyrethroid permethrin and the method of administering the composition in shampoo formulation to a warm blooded animal for subsequent rinse out, such that the active ingredients effectively kill existing parasites at the time of shampooing and after rinsing leave in the haircoat ovicidally effective amounts of pyriproxifen and insecticidally and acaricidally effective amounts of synthetic pyrethroid sufficient to protect the animal from reinfestation by ectoparasites and further to sterilize for an even longer period of time, ectoparasites that may survive the insecticidal and acaricidal activity of the synthetic pyrethroid. The compositions comprising the nitrogen containing heterocyclic ovicide and the insecticidally and acaricidally effective synthetic pyrethroid in the shampoo formulation, especially the most preferred composition of 0.01% to 0.5% of pyriproxifen and 1% to 2% of the synthetic pyrethroid permethrin and the method of administering the composition in shampoo formulation on the warm blooded animal such that the active ingredients effectively kill existing parasites at the time of shampooing and after rinsing leave in the haircoat ovicidally effective amounts of pyriproxifen and insecticidally and acaricidally effective amounts of synthetic pyrethroid sufficient to protect the animal from reinfestation by ectoparasites and further to sterilize for an even longer period of time, ectoparasites that may survive the insecticidal and acaricidal activity of the synthetic pyrethroid. Alternative embodiments comprise one or more active ingredients selected from the group consisting of permethrin, cypermethrin, cyhalothrin, lambdacyhalothrin, deltamethrin, tralomethrin, cyfluthrin, flucythrin, flumethrin, fluvalinate, fenvalerate, methoprene, fenoxycarb, benzoylphenyl urea, substituted benzoylphenyl urea, pyriproxifen, or nitrogen containing heterocyclics form the class defined above, in combination with a shampoo base comprising one or more of the above listed additives. Such compositions may also comprise one or more additional antiparasitic agents such as ivermectin.

It is expected that when any of the alternative compositions of the selected from the above combinations, as formulated in the examples 1, 2 and 3, are administered by shampooing the haircoat of dogs and cats followed by rinse out, which dogs and cats are infested with fleas and that are also maintained in an environment already infested with and favorable to the development of flea larvae, the pupation and emergence of new fleas to reinfest dogs and cats, and the dogs and cats are also reinfested by new ectoparasites including ticks, the animals will remain essentially free of both fleas and ticks for an extended period of time at the end of which, and for some time thereafter, new fleas will continue to be sterilized and treated animals will not suffer from continuing flea ant tick reinfestation whereas control dogs and cats will suffer severe and increasing infestations and reinfestations of both fleas and ticks. It is also expected that the insecticidal, acaricidal and ovicidal ectoparasitical compositions will show broad protection against fleas, ticks, mosquitoes, lice, mange mites and biting flies. It is anticipated that combinations of pyriproxifen and halogen containing synthetic pyrethroids, such as permethrin and related compounds, will also exhibit enhanced synergistic effects wherein one or more compositions of the mixtures is more efficacious than the same dose administered alone. As used herein "long lasting" or "long-acting" means a residual activity which keeps the treated animal substantially protected against ectoparasites for at least ten days following treatment.

The synthetic pyrethroids may also be combined with a synergist to enhance the toxicity of the pyrethroid towards insects. The chemical names of certain of the suitable pyrethroids and synergists are listed below.

Illustrative synthetic pyrethroids (with common industrial names) :
3-phenoxyphenylmethyl
3-(2,2-dichloroethenyl)-2,2-dimethylcyclopropane carboxylate (Permethrin) ;
(+)-cyano-(3-phenoxyphenyl)methyl (+)-cis-trans-3 (2,2-dichloroethenyl)-2,2-dimethylcyclopropane carboxylate (Cypermethrin) ;
alpha-cyano-3-phenoxybenzyl-d,cis-dibromochrysanthemate (Deltamethrin) ;
(RS)-alpha-cyano-3-phenoxybenzyl (1RS)-cis-3-((Z)-2-chloro-3,3,3,trifluoroprop-1-enyl)-2,2-dimethyl cyclopropane-carboxylate (Cyhalothrin) ;
(R+S)cyano-3-phenoxybenzyl (1S+1R)-cis-3-(Z-2-chloro-3,3,3,trifluoroprop-1-enyl)-2,2-dimethylcyclopropane carboxylate (Lambdacyhalothrin) ;
cyano(3-phenoxyphenyl)methyl 2,2-dimethyl-3-(1,2,2,2-tetrabromoethyl)cyclopropanecarboxylate (Tralomethrin) ;
cyano(4-fluoro-3-phenoxyphenyl)methyl 3-(2,2-dichloro ethenyl)-2,2-dimethylcyclopropane carboxylate (Cyfluthrin);
cyano(3-phenoxyphenyl)methyl 2-(4-difluoromethoxyphenyl) isovalerate (Flucythrinate) ;
cyano(4-fluoro-3-phenoxyphenyl)methyl 3-[2-chloro-2-(4-chlorophenyl)ethenyl)-2,2-dimethylcyclopropane carboxylate (Flumethrin);
cyano(3-phenoxyphenyl)methyl 2-(4-chlorophenyl) isovalerate (Fenvalerate).

### Illustrative Synergists

### Piperonyl butoxide

N-octyl-bicycloheptene dicarboximide organic thiocyanates in which the organic group is a long aliphatic organic radical having between 8 and 14 carbon atoms such as octyl thiocyanate, nonyl thiocyanate, decyl thiocyanate and undecenyl thiocyanate octachlorodipropyl ether.

The following examples illustrate the invention described herein, but do not limit its scope in any way.

### Example 1 : Pyriproxifen Residual Shampoo for Dogs and Cats

Flea sterilizing shampoo containing 0.25% pyriproxifen, designed to be applied to dogs or cats at the dose rates, respectively, of 7.5 g and 12 g shampoo per kg bodyweight, that will provide, after complete rinse-off, continuing ovicidal/flea sterilizing effect for three to four months.

| Batch of 1000 kg | | | |
|---|---|---|---|
| | | | |
| Composition | Potency | % | Amount (kg) |
| Pyriproxifen technical @ | 93% | 0.25% | 2.69 |
| Ethanol | | 5.00% | 50.00 |
| Petroleum distillate | | 0.50% | 5.00 |
| Ammonium lauryl sulfate | | 40.00% | 400.00 |
| Alkanolamide maleic acid | | 15.00% | 150.00 |
| Fragrance | | 0.50% | 5.00 |
| Deionized water | | 38.73% | 387.31 |

The ingredients are blended together in the sequence shown, pH adjusted to 6.5 +/- 0.25 with lactic acid and viscosity adjusted with sodium chloride to the range of 2000 cps at 26°C to 3000 cps at 22°C, then filled into high density polyethylene bottles with flip top dispensing caps. This shampoo is designed to be used on dogs or cats that are not presently infested with fleas but that are in an environment where reinfestation is a seasonal risk. Repeated treatment will be necessary every three to four months, or more frequently for cosmetic cleansing, and should continue as long as an infestation risk persists. The pyriproxifen is designed to prevent any environmental contamination with fertile flea eggs should the pet be accidentally infested. This will interrupt the flea life cycle such that the environment will remain free of fleas when the shampoo is used regularly. Alternatively, methoprene, fenoxycarb or benzoylphenyl urea, or another nitrogen containing heterocyclic within the class defined above may be substituted for pyriproxifen in a concentration proportional to the relative potency of the selected materials.

### Example 2 : Pyriproxifen and Permethrin Residual Emollient Shampoo for Dogs or Cats

Flea sterilizing and flea and tick killing emollient shampoo containing 0.1% pyriproxifen and 0.5% permethrin, designed to be applied to dogs or cats at the respective rates of 7.5 g and 10 g shampoo per kg bodyweight, that will kill attached fleas and ticks and, after complete rinse off, will provide residual protection against new flea and tick reinfestation for about ten days and then a continuing ovicidal/flea sterilizing effect for a further two or three months.

Alternatively, methoprene, fenoxycarb or benzoylphenyl urea, or another nitrogen containing heterocyclic within the class defined above may be substituted for pyriproxifen and cypermethrin, cyhalothrin, lambdacyhalothrin, deltamethrin, tralomethrin, cyfluthrin, flucythrin, flumethrin, fluvalinate or fenvalerate may be substituted for permethrin, in each case being substituted in a concentration proportional to the relative residual potency of the selected materials, as is easily determined by one skilled in the formulation art.

| Batch of 1000kg | | | |
|---|---|---|---|
| | | | |
| Composition | Potency | % | Amount (kg) |
| Pyriproxifen technical @ | 93% | 0.10% | 1.08 |
| Permethrin technical @ | 96% | 0.50% | 5.21 |
| Ethanol | | 5.00% | 50.00 |
| Oat protein | | 2.00% | 20.00 |
| Lanolin | | 0.20% | 2.00 |
| PEG 80 sorbitan laureate | | 5.10% | 51.00 |
| Sodium trideceth sulfate | | 4.50% | 45.00 |
| Cocamidopropylhydroxysultane | | 3.48% | 34.80 |
| PEG 150 distearate | | 1.95% | 19.50 |
| Lauroamphocarboxyglycinate | | 3.00% | 30.00 |
| Sodium laureth-13 carboxylate | | 0.60% | 6.00 |
| Lauramine oxide | | 2.00% | 20.00 |
| Fragrance | | 0.30% | 3.00 |
| Preservative | | 1.00% | 10.00 |
| Deionized water | | 70.24% | 702.42 |

### Example 3 : Pyriproxifen and Permethrin Residual Shampoo for Dogs

Flea sterilizing and flea and tick killing shampoo containing 0.25% pyriproxifen and 1.0% permethrin, designed to be applied to dogs at the rate of 7.5 g shampoo per kg bodyweight, that will kill all attached fleas and ticks and, after complete rinse off, will provide residual protection against new flea and tick reinfestation for about three weeks and then a continuing ovicidal/flea sterilizing effect for a further three to four months.

Alternatively, methoprene, fenoxycarb or a benzoylphenyl urea, or another nitrogen containing heterocyclic within the class defined above may be substituted for pyriproxifen and cypermethrin, cyhalothrin, lambdacyhalothrin, deltamethrin, tralomethrin, cyfluthrin, flucythrin, flumethrin, fluvalinate or fenvalerate may be substituted for permethrin, in each case being substituted in a concentration proportional to the relative residual potency of the selected materials, as is easily determined by one skilled in the formulation art.

| Batch of 1000 kg | | | |
|---|---|---|---|
| | | | |
| Composition | Potency | % | Amount (kg) |
| Pyriproxifen technical @ | 93% | 0.25% | 2.69 |
| Permethrin technical @ | 96% | 1.00% | 10.42 |
| Ethanol | | 5.00% | 50.00 |
| Ammonium lauryl sulfate | | 40.00% | 400.00 |
| Alkanolamide maleic acid | | 15.00% | 150.00 |
| Fragrance | | 0.50% | 5.00 |
| Deionized water | | 43.19% | 381.90 |

The ingredients are blended together in the sequence shown, pH adjusted to 6.5 +/- 0.25 with lactic acid and viscosity adjusted with sodium chloride to the range of 2000 cps at 26°C to 3000 cps at 22°C, then filled into high density polyethylene bottles with flip top dispensing caps. This shampoo is designed to be used on dogs or cats that are initially infested with fleas and ticks and that are in an environment where reinfestation is continuing. Repeated treatment will be necessary every one to two weeks as long as the reinfestation risk persists. The pyriproxifen component is designed to prevent environmental contamination with new flea eggs and thus will interrupt the flea life cycle such that the environment will be free of new fleas by three to six months after first using the shampoo.

### Example 4 : Permethrin Residual Shampoo for Dogs and Cats

Flea sterilizing shampoo containing 1.0% permethrin, designed to be applied to dogs or cats at the dose rates, respectively, of 7.5 g and 10 g shampoo per kg bodyweight, that will provide immediate efficacy against existing burdens of fleas and ticks and, after complete rinse-off, continuing residual protection against reinfestation with new fleas and new ticks for two or three weeks, in absence of any further insecticidal or acaricidal treatments. Alternatively cypermethrin, cyhalothrin, lambdacyhalothrin, deltamethrin, tralomethrin, cyfluthrin, flucythrin, flumethrin, fluvalinate or fenvalerate may be substituted for permethrin, in each case being substituted in a concentration proportional to the relative residual potency of the selected material, as is easily determined by one skilled in the formulation art.

| Batch of 1000kg | | | |
|---|---|---|---|
| | | | |
| Composition | Potency | % | Amount (kg) |
| Permethrin technical @ | 96% | 1.00% | 10.42 |
| Ethanol | | 2.50% | 25.00 |
| Polysorbate 20 | | 2.00% | 20.00 |
| Ammonium lauryl sulfate | | 30.00% | 300.00 |
| Alkanolamide maleic acid | | 10.00% | 100.00 |
| Fragrance | | 0.80% | 8.00 |
| Deionized water | | 53.66% | 536.58 |

The ingredients are blended together in the sequence shown, pH adjusted to 6.5 +/- 0.25 with citric acid and viscosity adjusted with sodium chloride to the range of 1000 cps at 26°C to 1500 cps at 22°C, then filled into high density polyethylene bottles with flip top dispensing caps. This shampoo is designated to be used on dogs or cats that are infested with fleas and ticks and are in an environment where reinfestation is a continuing ever-present risk. Repeated treatment will be necessary every two to four weeks, depending on the reinfestation pressure, or more frequently for cosmetic cleansing, and should continue as long as an infestation risk persists. The pet will remain essentially free of fleas and ticks when the shampoo is used regularly.

### Example 5 : Immediate and Residual Efficacy of a Permethrin Shampoo against Fleas and Ticks on Dogs and Cats

A permethrin shampoo was formulated in accordance with Example 4 to contain 1.0% of technical permethrin. Twelve cats and twelve dogs were selected, randomized into four groups, two each of six dogs and two of six cats. The dogs were infested each with 100 *Ctenocephalides felis* fleas and 25 ticks each of two species, the Brown Dog Tick, *Rhipicephalus sanguineous* and the American Dog Tick, *Dermacentor variabilis.* The cats were each infested with 100 fleas. The following day six dogs and six cats were treated by wetting their haircoats thoroughly, applying shampoo at rates of 7.5 and 10 g/kg, and hence applying a residual insecticidally effective amount of permethrin to the animals' haircoats at the mean group rates of 72.5 and 100.8 mg/kg, respectively, lathering thoroughly with additional water as needed to effect good coverage, allowing to stand for five minutes then rinsing off thoroughly with water and air drying without towelling. Fleas and ticks washed off in the rinse water were counted. The other six dogs and cats were not treated but served as controls. Flea and tick counts were made on all animals 24 and 72 hours later, and the animals were reinfected, as previously, with fleas and ticks. There were no further applications of shampoo or any other treatment that would have a deleterious effect on fleas and ticks on the animals. Flea and tick counts were again made 24 and 72 hours after reinfection. This cycle of reinfestation and enumeration was repeated five times more until residual efficacy was considered to be inadequate (i.e., less than 80% group mean reduction in ectoparasite burdens in treated animals compared with mean group control animal burden).

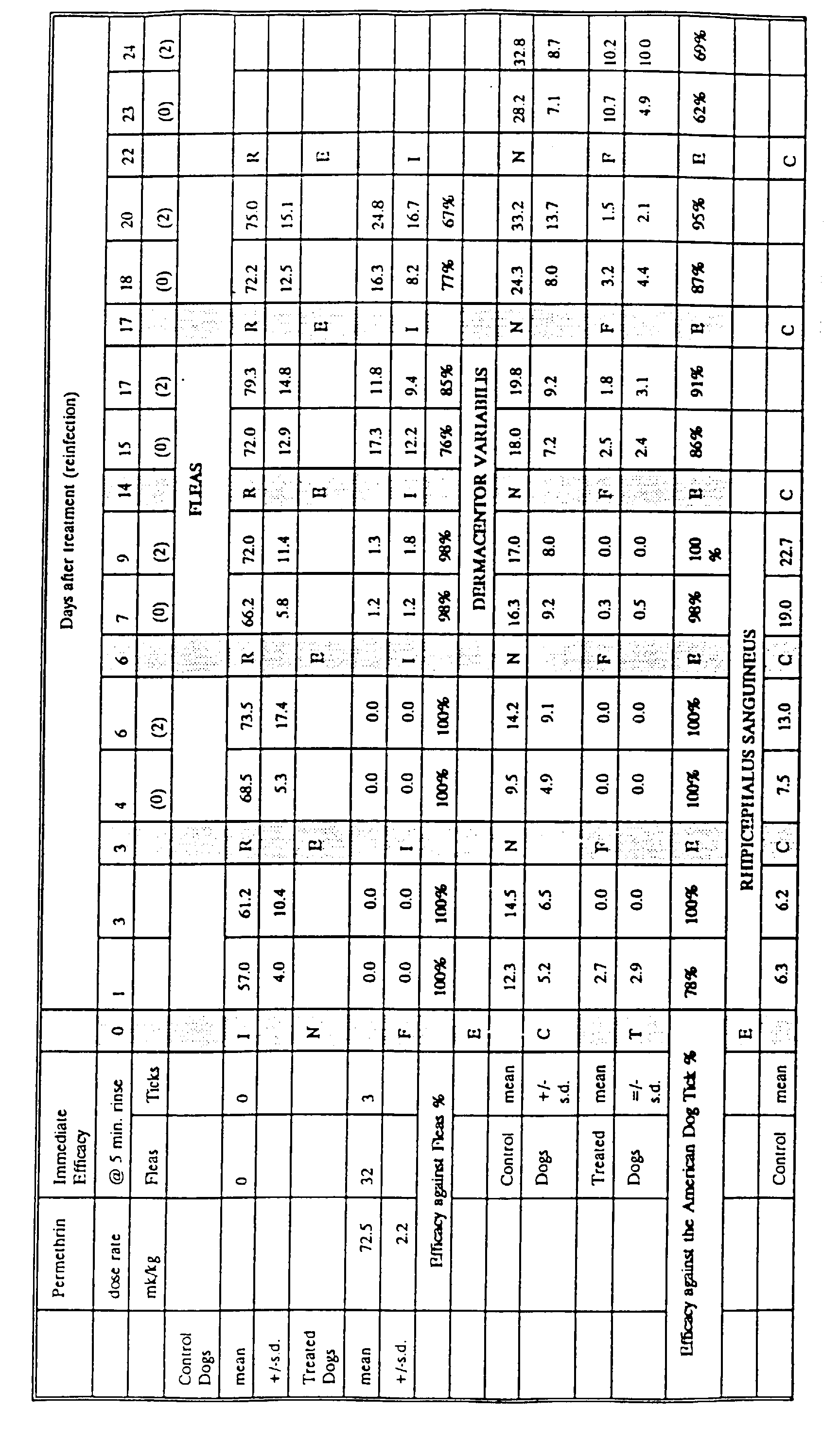

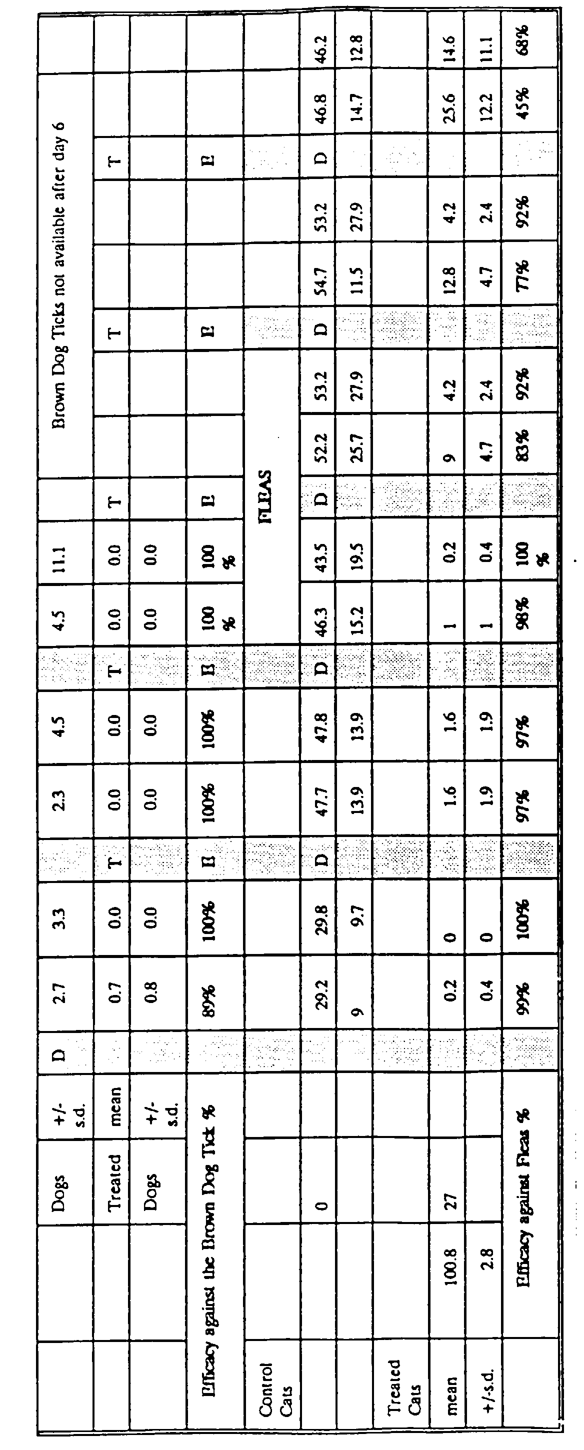

Immediate efficacy of the shampoo was shown by the loss of dead fleas and ticks in the rinse water, followed by 78 to 100% reduction in mean ectoparasite burdens in the treated animals the following day and 100% efficacy 48 hours later. This immediate efficacy is as expected. However, the continuing residual efficacy against five repeated reinfestations with new fleas and ticks for the following three weeks was totally unexpected since rinsing out the animals' haircoats has heretofore been found to remove the active ingredients of shampoos and to leave the treated animal fully susceptible to reinfestation immediately thereafter.

### Example 6 : Residual Ovicidal Efficacy of a Pyriproxifen Shampoo against Fleas on Cats

Three pyriproxifen shampoos were formulated in accordance with Example 1 to contain 0.01%, 0.05% and 0.25% of technical pyriproxifen. Twenty cats were selected and randomized into four groups, each of five cats. The cats were infested each with 100 *Ctenocephalides felis* fleas. Flea eggs were collected (50/cat) and incubated in nutrient medium under suitable conditions of temperature and humidity. After 24 hours the numbers of hatched eggs were counted and the larvae returned to the medium for a further 28 days, after which time the numbers of emerged fleas were enumerated. From these data the normal flea egg fertility rate was determined and the 20 cats were judged to be good hosts for the maintenance of fertile fleas.

Fifteen cats in three groups, each of five cats, served as principals and were treated with one of the three pyriproxifen shampoos. Their haircoats were thoroughly wetted with water, shampoo was applied at the rate of 10 g/kg and their coats were lathered thoroughly with additional water, as needed to effect good coverage. The cats were allowed to stand for five minutes then rinsed off thoroughly with water and air dried without towelling. The remaining five cats served as controls and were similarly treated by shampooing with a control shampoo that was formulated to be the same as the active shampoos but without pyriproxifen. The cats were reinfested with fleas frequently, at least weekly. Flea eggs were collected at least weekly and incubated. The egg/larva cultures were examined at 4 days and 28 days when egg hatch and adult flea emergence values, respectively, were determined. There were no further applications of shampoo nor any other treatment that would have a deleterious effect on the fleas on the cats. The cycle of reinfestation and egg collection was repeated until residual ovicidal efficacy/flea egg sterilization from the principal cats was considered to be inadequate (i.e., less than 80% group mean reduction in egg hatch/adult flea emergence in treated animals compared with mean group flea egg fertility values from the control cats) or, in absence in treatment failure, at approximately three months after treatment.

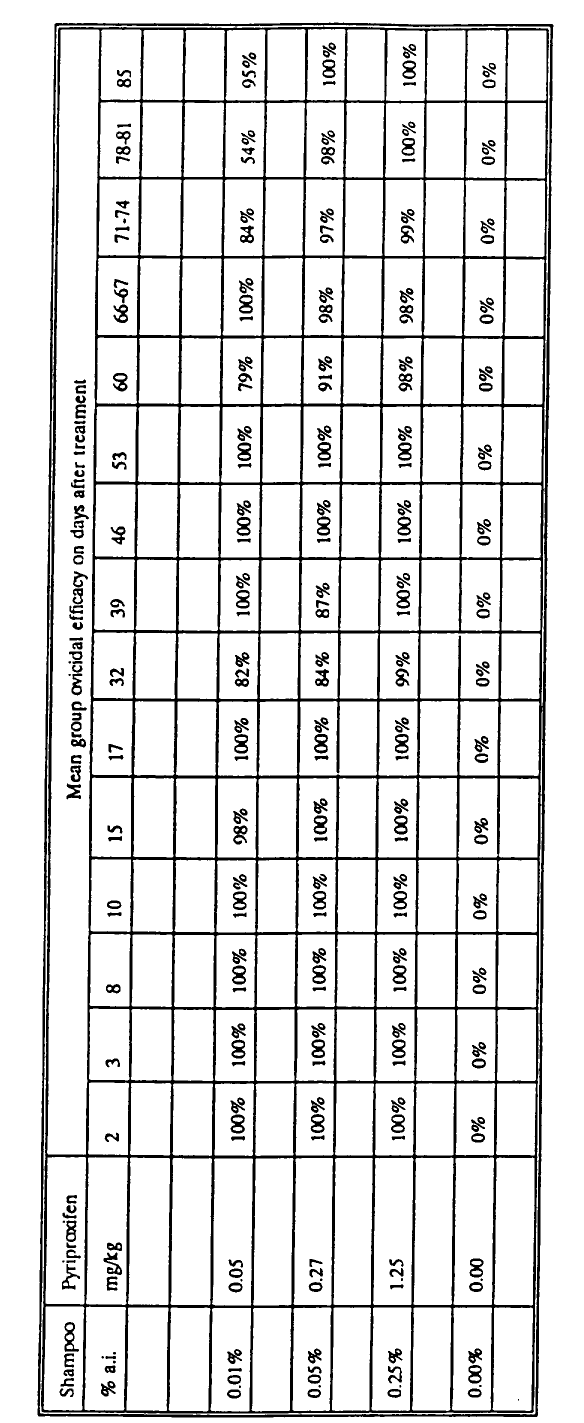

Immediate ovicidal/flea egg sterilization efficacy of the shampoo on the treated cats was shown by the complete sterilization of all fleas (both pre-existing and those applied immediately after the cats had dried) by 48 hours after shampooing. It was also noted that the active ingredient, pyriproxifen, although known primarily for its ovicidal effect was, at the higher levels also insecticidal since it was difficult to maintain adequate flea burdens for egg collection on those treated cats for at least the first month after shampooing. Residual ovicidal efficacy that sterilized all fleas, both pre-existing and from periodic new reinfestations, continued for up to and beyond 85 days, in spite of the thorough post-shampoo rinsing of the cats' haircoats to wash out the active ingredient of the shampoo.

## Claims

1. Use of a detergent and of at least one ovicidally active compound selected from the nitrogen containing heterocyclic compounds of the formula : 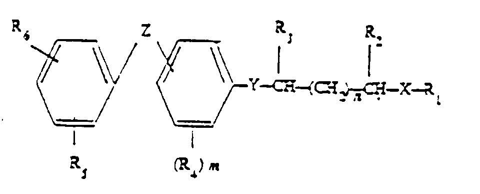 wherein :
- R₁ is one of the following groups : 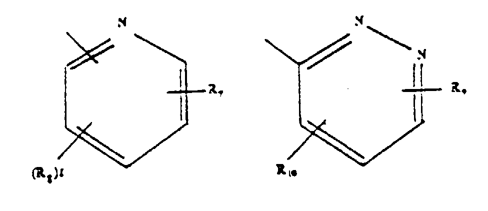 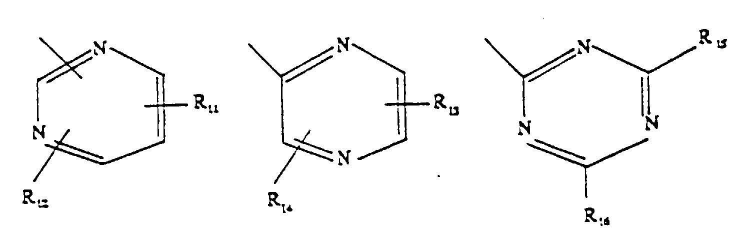 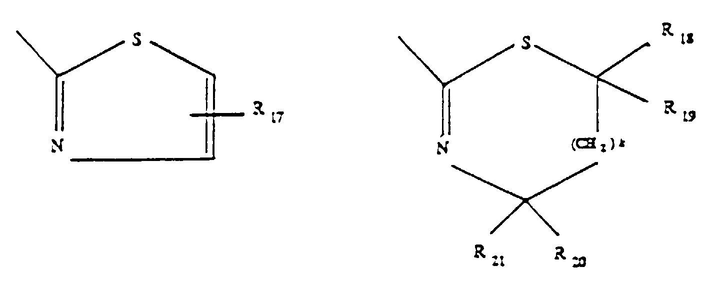 in which R₇, R₈, R₉, R₁₀, R₁₁, R₁₂, R₁₃, R₁₄, R₁₅, R₁₆ and R₁₇ are, the same or different, each a hydrogen atom, a halogen atom, a C₁-C₄ alkoxy group, a C₁-C₄ alkylthiogroup, a trifluoro methyl group or a nitro group ; R₁₈, R₁₉, R₂₀ and R₂₁ are, the same or different, each a hydrogen atom or a methyl group, *k* is an integer of 0 to 1 and *l* is an integer of 0 to 3 ;
- R₂ and R₃ are, the same or different, each a hydrogen atom, a halogen atom or a methyl group ;
- R₄ is a halogen atom or a methyl group ;
- R₅ and R₆ are, the same or different, each a hydrogen atom, a halogen atom, a C₁-C₄ haloalkyl group or a C₁-C₄ haloalkoxy group ;
- X, Y and Z are, the same or different, each an oxygen atom, a sulfur atom or a methylene group, *m* is an integer of 0 to 4 and *n* is an integer of 0 to 2;
for the preparation of a long-acting composition for the residual control of ectoparasites which attack warm-blooded animals, said composition being under the form of a shampoo designed to be rinsed out, and comprising a dose of active compound sufficient for leaving on the haircoat of said warm-blooded animals, after rinsing out, an ovicidally residual effective amount of said active compound.

2. Use according to claim 1, wherein the shampoo comprises an ovicidally active compound which is a nitrogen containing heterocyclic compound in which R1 is a 2-substituted pyridine.

3. Use according to claim 2, wherein the nitrogen containing heterocyclic compound is a (4-phenoxyphenoxy)ethoxy pyridine and more preferably the 2-[1-methyl-2-(4-phenoxyphenoxy)ethoxy] pyridine (pyriproxifen).

4. Use according to anyone of claims 1 to 3, wherein the dose of the ovicidally active compound in the shampoo composition is in the range from 0.001% to 5%, more preferably from 0.005% to 2.5% and most preferably from 0.01% to 0.5%.

5. Use according to anyone of claims 1 to 4, wherein the shampoo composition further comprises an insecticidally and acaracidally active compound which is a synthetic pyrethroid selected from the group consisting of permethrin, cypermethrin, cyhalothrin, lambda-cyhalothrin, deltamethrin, tralomethrin, cyfluthrin, flucythrin, flumethrin, fluvalinate, fenvalerate or mixtures thereof, and leaves on the haircoat of the warm-blooded animals, after rinsing out, an ovicidally residual effective amount of said nitrogen containing heterocyclic compound and an insecticidally and acaricidally residual effective amount of said synthetic pyrethroid.

6. Use according to claim 5, wherein the dose of the synthetic pyrethroid in the shampoo composition is in the range from 0.2 to 5.0%, more preferably from 0.5 to 3.0% and most preferably from 1.0 to 2.0%.

7. Use according to claim 5, wherein the nitrogen containing heterocyclic compound is the 2-[1-methyl-2-(4-phenoxyphenoxy)ethoxy] pyridine (pyriproxifen) whereas the synthetic pyrethroid is the permethrin.

8. Use according to claim 5 or claim 7, wherein the dose of the nitrogen containing heterocyclic compound in the shampoo composition is in the range from 0.001% to 5% whereas the dose of the synthetic pyrethroid in the shampoo composition is in the range from 0.2% to 5.0%.

9. Use according to claim 5 or claim 7, wherein the dose of the nitrogen containing heterocyclic compound in the shampoo composition is in the range from 0.005% to 2.5% whereas the dose of the synthetic pyrethroid in the shampoo composition is in the range from 0.5% to 3.0%.

10. Use according to claim 5 or to claim 7, wherein the dose of the nitrogen containing heterocyclic compound in the shampoo composition is in the range from 0.01% to 0.5% whereas the dose of the synthetic pyrethroid in the shampoo composition is in the range from 1.0% to 2.0%.

11. Use according to anyone of claims 1 to 10, wherein the shampoo composition contains from 1.0% to 20.0% emulsifier in combination with 10% to 60% detergent and optionally one or more components selected from the group consisting of emollients, fragances, preservatives and viscosity controlling agents.

12. Use according to anyone of claims 1 to 11, wherein the ectoparasites are fleas or ticks and the warm-blooded animal is a dog or a cat.

## Patentansprüche

1. Verwendung eines Detergens und mindestens einer ovizid wirkenden Verbindung, ausgewählt aus den stickstoffhaltigen heterocyclischen Verbindungen der Formel: 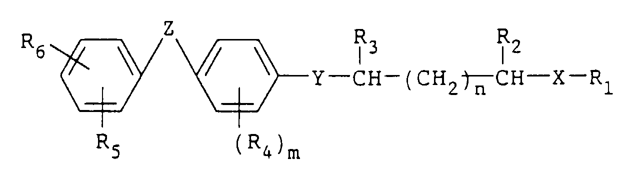 worin:
- R₁ eine der folgenden Gruppen ist: 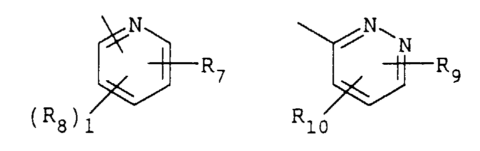 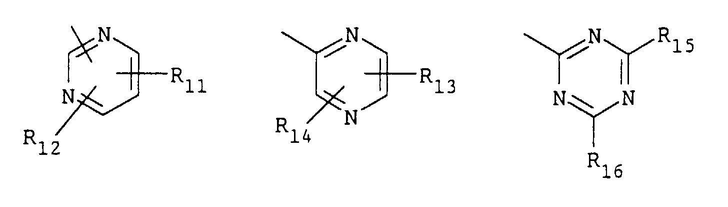 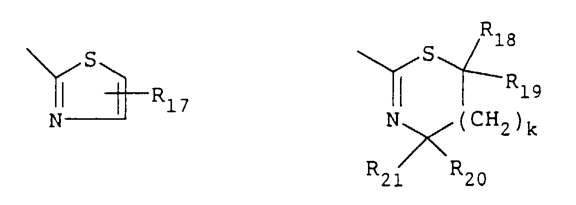 worin R₇, R₈, R₉, R₁₀, R₁₁, R₁₂, R₁₃, R₁₄, R₁₅, R₁₆ und R₁₇ gleich oder verschieden jeweils ein Wasserstoffatom, ein Halogenatom, eine C₁-C₄-Alkoxygruppe, eine C₁-C₄-Alkylthiogruppe, eine Trifluormethylgruppe oder eine Nitrogruppe sind; R₁₈, R₁₉, R₂₀ und R₂₁ jeweils ein Wasserstoffatom oder eine Methylgruppe sind, k eine ganze Zahl von 0 bis 1 ist und 1 eine ganze Zahl von 0 bis 3 ist;
- R₂ und R₃ gleich oder verschieden jeweils ein Wasserstoffatom, ein Halogenatom oder eine Methylgruppe sind;
- R₄ ein Halogenatom oder eine Methylgruppe ist;
- R₅ und R₆ gleich oder verschieden jeweils ein Wasserstoffatom, ein Halogenatom, eine C₁-C₄-Halogenalkylgruppe oder eine C₁-C₄-Halogenalkoxygruppe sind;
- X, Y und Z gleich oder verschieden jeweils ein Sauerstoffatom, ein Schwefelatom oder eine Methylengruppe sind, m eine ganze Zahl von 0 bis 4 ist und n eine ganze Zahl von 0 bis 2 ist;
zur Herstellung einer lang wirkenden Zusammensetzung zur Restbekämpfung von Ektoparasiten, die warmblütige Tiere befallen, wobei die Zusammensetzung in der Form eines Shampoos vorliegt, das zum Ausspülen entworfen ist und eine Dosis einer aktiven Verbindung besitzt, die ausreichend ist, so daß nach dem Ausspülen eine ovizid wirkende Restmenge der aktiven Verbindung auf dem Haarpelz der warmblütigen Tiere verbleibt.

2. Verwendung nach Anspruch 1, wobei das Shampoo eine ovizid wirkende Verbindung umfaßt, die eine stickstoffhaltige heterocyclische Verbindung ist, in der R₁ ein 2-substituieres Pyridin ist.

3. Verwendung nach Anspruch 2, wobei die stickstoffhaltige heterocyclische Verbindung ein (4-Phenoxyphenoxy)ethoxypyridin ist und weiter bevorzugt das 2-[1-Methyl-2-(4-phenoxyphenoxy)ethoxy]pyridin (Pyriproxifen) ist.

4. Verwendung nach einem der Ansprüche 1 bis 3, wobei die Dosis der ovizid wirkenden Verbindung in der Shampoo-Zusammensetzung im Bereich von 0,001% bis 5%, weiter bevorzugt von 0,005% bis 2,5% und am meisten bevorzugt von 0,01% bis 0,5% liegt.

5. Verwendung nach einem der Ansprüche 1 bis 4, wobei die Shampoo-Zusammensetzung ferner eine insektizid und akarizid wirkende Verbindung umfaßt, welche ein synthetisches Pyrethroid ist, das aus der Gruppe ausgewählt ist, bestehend aus Permethrin, Cypermethrin, Cyhalothrin, lambda-Cyhalothrin, Deltamethrin, Tralomethrin, Cyfluthrin, Flucythrin, Flumethrin, Fluvalinat, Fenvalerat oder deren Mischungen, und nach dem Ausspülen eine ovizid wirkende Restmenge der stickstoffhaltigen heterocyclischen Verbindung und eine insektizid und akarizid wirkende Restmenge des synthetischen Pyrethroids auf dem Haarpelz der warmblütigen Tieren verbleibt.

6. Verwendung nach Anspruch 5, wobei die Dosis des synthetischen Pyrethroids in der Shampoo-Zusammensetzung im Bereich von 0,2 bis 5,0%, weiter bevorzugt von 0,5 bis 3,0% und am meisten bevorzugt von 1,0 bis 2,0% liegt.

7. Verwendung nach Anspruch 5, wobei die stickstoffhaltige heterocyclische Verbindung das 2-[1-Methyl-2-(4-phenoxyphenoxy)ethoxy]pyridin (Pyriproxifen) ist, während das synthetische Pyrethroid das Permethrin ist.

8. Verwendung nach Anspruch 5 oder Anspruch 7, wobei die Dosis der stickstoffhaltigen heterocyclischen Verbindung in der Shampoo-Zusammensetzung im Bereich von 0,001% bis 5% liegt, während die Dosis des synthetischen Pyrethroids in der Shampoo-Zusammensetzung im Bereich von 0,2% bis 5,0% liegt.

9. Verwendung nach Anspruch 5 oder Anspruch 7, wobei die Dosis der stickstoffhaltigen heterocyclischen Verbindung in der Shampoo-Zusammensetzung im Bereich von 0,005% bis 2,5% liegt, während die Dosis des synthetischen Pyrethroids in der Shampoo-Zusammensetzung im Bereich von 0,5% bis 3,0% liegt.

10. Verwendung nach Anspruch 5 oder Anspruch 7, wobei die Dosis der stickstoffhaltigen heterocyclischen Verbindung in der Shampoo-Zusammensetzung im Bereich von 0,01% bis 0,5% liegt, während die Dosis des synthetischen Pyrethroids in der Shampoo-Zusammensetzung im Bereich von 1,0% bis 2,0% liegt.

11. Verwendung nach einem der Ansprüche 1 bis 10, wobei die Shampoo-Zusammensetzung von 1,0% bis 20,0% eines Emulgators in Kombination mit 10% bis 60% eines Detergens und gegebenenfalls eine oder mehrere Komponenten enthält, die aus der Gruppe ausgewählt sind, bestehend aus Aufweichmitteln, Düften, Konservierungsstoffen und viskosizitätssteuernden Mitteln.

12. Verwendung nach einem der Ansprüche 1 bis 11, wobei die Ektoparasiten Flöhe oder Zecken sind und das warmblütige Tier ein Hund oder eine Katze ist.

## Revendications

1. Utilisation d'un détergent et d'au moins un composé actif du point de vue ovicide choisi parmi les composés hétérocycliques contenant de l'azote de formule : 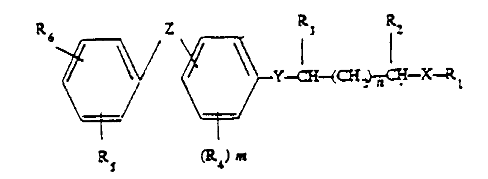 dans laquelle :
- R₁ est l'un des groupes suivants : 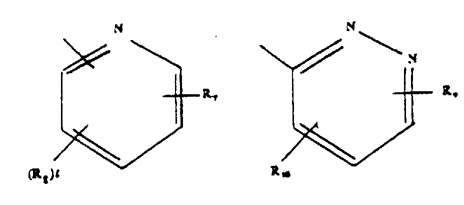 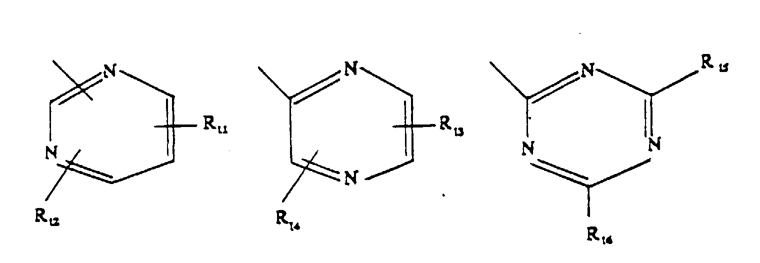 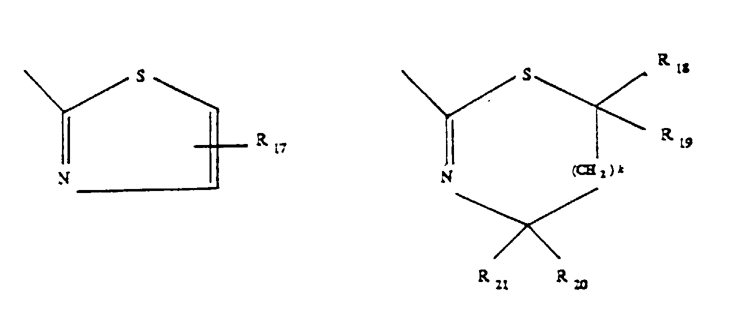 où R₇, R₈, R₉, R₁₀, R₁₁, R₁₂, R₁₃, R₁₄, R₁₅, R₁₆ et R₁₇ sont identiques ou différents, et sont chacun un atome d'hydrogène, un atome d'halogène, un groupe alcoxy en C₁₋₄, un groupe alkylthio en C₁₋₄, un groupe trifluorométhyle ou un groupe nitro ; R₁₈, R₁₉, R₂₀ et R₂₁ sont identiques ou différents, et sont chacun un atome d'hydrogène ou un groupe méthyle, k est un entier de 0 à 1 et 1 est un entier de 0 à 3 ;
- R₂ et R₃ sont identiques ou différents, et sont chacun un atome d'hydrogène, un atome d'halogène ou un groupe méthyle ;
- R4 est un atome d'halogène ou un groupe méthyle ;
- R5 et R₆ sont identiques ou différents, et sont chacun un atome d'hydrogène, un atome d'halogène, un groupe haloalkyle en C₁₋₄ ou un groupe haloalcoxy en C₁₋₄ ;
- X, Y et Z sont identiques ou différents, et sont chacun un atome d'oxygène, un atome de soufre ou un groupe méthylène, m est un entier de 0 à 4 et n est un entier de 0 à 2 ;
pour la préparation d'une composition à longue durée pour l'élimination rémanente d'ectoparasites qui attaquent les animaux à sang chaud, ladite composition étant sous la forme d'un shampooing destiné à être éliminé par rinçage, et comprenant une dose de composé actif suffisante pour laisser sur le pelage desdits animaux à sang chaud, après rinçage, une quantité résiduelle efficace du point de vue ovicide dudit composé actif.

2. Utilisation suivant la revendication 1, dans laquelle le shampooing comprend un composé actif du point de vue ovicide qui est un composé hétérocyclique contenant de l'azote dans lequel R₁ est une pyridine 2-substituée.

3. Utilisation suivant la revendication 2, dans laquelle le composé hétérocyclique contenant de l'azote est une (4-phénoxyphénoxy)-éthoxy-pyridine et plus avantageusement la 2-[1-méthyl-2-(4-phénoxyphénoxy)-éthoxy]-pyridine (pyriproxifène).

4. Utilisation suivant l'une quelconque des revendications 1 à 3, dans laquelle la dose du composé actif du point de vue ovicide dans la composition de shampooing est comprise dans la gamme de 0,001% à 5%, de préférence de 0,005% à 2,5% et le plus avantageusement de 0,01% à 0,5%.

5. Utilisation suivant l'une quelconque des revendications 1 à 4, dans laquelle la composition de shampooing comprend de plus un composé actif du point de vue insecticide et acaricide qui est un pyréthroïde de synthèse choisi dans le groupe consistant en perméthrine, cyperméthrine, cyhalothrine, lambda-cyhalothrine, deltaméthrine, tralométhrine, cyfluthrine, flucythrine, fluméthrine, fluvalinate, fenvalérate ou mélanges de ceux-ci, et laisse sur le pelage des animaux à sang chaud, après rinçage, une quantité résiduelle efficace du point ovicide dudit composé hétérocyclique contenant de l'azote et une quantité résiduelle efficace du point de vue insecticide et acaricide dudit pyréthroïde de synthèse.

6. Utilisation suivant la revendication 5, dans laquelle la dose du pyréthroïde de synthèse dans la composition de shampooing est comprise dans la gamme de 0,2 à 5,0%, de préférence de 0,5 à 3,0% et plus avantageusement de 1,0 à 2,0%.

7. Utilisation suivant la revendication 5, dans laquelle le composé hétérocyclique contenant de l'azote est la 2-[1-méthyl-2-(4-phénoxyphénoxy)-éthoxy]-pyridine (pyriproxifène), tandis que le pyréthroïde de synthèse est la perméthrine.

8. Utilisation suivant les revendications 5 ou 7, dans laquelle la dose du composé hétérocyclique contenant de l'azote dans la composition de shampooing est comprise dans la gamme de 0,001% à 5%, tandis que la dose du pyréthroïde de synthèse dans la composition de shampooing est comprise dans la gamme de 0,2% à 5,0%.

9. Utilisation suivant les revendications 5 ou 7, dans laquelle la dose du composé hétérocyclique contenant de l'azote dans la composition de shampooing est comprise dans la gamme de 0,005% à 2,5%, tandis que la dose du pyréthroïde de synthèse dans la composition de shampooing est comprise dans la gamme de 0,5% à 3,0%.

10. Utilisation suivant les revendications 5 ou 7, dans laquelle la dose du composé hétérocyclique contenant de l'azote dans la composition de shampooing est comprise dans la gamme de 0,01% à 0,5%, tandis que la dose du pyréthroïde de synthèse dans la composition de shampooing est comprise dans la gamme de 1,0% à 2,0%.

11. Utilisation suivant l'une quelconque des revendications 1 à 10, dans laquelle la composition de shampooing comprend de 1,0% à 20,0% d'émulsifiant en combinaison avec 10% à 60% de détergent et éventuellement un ou plusieurs composants choisis dans le groupe consistant en adoucissants, parfums, conservateurs et agents de contrôle de la viscosité.

12. Utilisation suivant l'une quelconque des revendications 1 à 11, dans laquelle les ectoparasites sont les puces ou les tiques et l'animal à sang chaud est un chien ou un chat.
